# EUROPEAN PATENT SPECIFICATION

(11) **EP 4 213 765 B1**
(45) Date of publication and mention of the grant of the patent: **04.11.2026**
(21) Application number: 20786185.7
(22) Date of filing: 21.09.2020
(51) Int. Cl.: A61F 2/16

(54) **INTRAOCULAR LENS**
INTRAOKULARLINSE
LENTILLE INTRAOCULAIRE

(43) Date of publication of application: 26.07.2023
(73) Proprietor: Carl Zeiss Meditec AG, 07745 Jena (DE)
(72) Inventor: SCHREIBER, Benjamin, 73447 Oberkochen (DE); BADUR, Thorben, 73447 Oberkochen (DE)
(74) Representative: PATERIS PartmbB
(86) International application number: PCT/US2020/051793
(87) International publication number: WO 2022/060371

(56) References cited:
- EP-A2- 1 462 071
- DE-A1- 102011 053 670
- BULL JAMES N. ET AL: "From E to Z and back again: reversible photoisomerisation of an isolated charge-tagged azobenzene", PHYSICAL CHEMISTRY CHEMICAL PHYSICS, vol. 20, no. 1, 1 January 2018 (2018-01-01), pages 509 - 513, XP055809255, ISSN: 1463-9076, DOI: 10.1039/C7CP07278C
- BUSHUYEV OLEKSANDR S. ET AL: "Photo-mechanical azobenzene cocrystals and in situ X-ray diffraction monitoring of their optically-induced crystal-to-crystal isomerisation", CHEMICAL SCIENCE, vol. 5, no. 8, 1 January 2014 (2014-01-01), United Kingdom, pages 3158 - 3164, XP055809322, ISSN: 2041-6520, Retrieved from the Internet <URL:https://pubs.rsc.org/en/content/articlepdf/2014/sc/c4sc00987h> DOI: 10.1039/C4SC00987H
- KIM DAE-YOON ET AL: "Light responsive liquid crystal soft matters: structures, properties, and applications", LIQUID CRYSTALS TODAY, vol. 28, no. 2, 3 April 2019 (2019-04-03), GB, pages 34 - 45, XP055809307, ISSN: 1358-314X, DOI: 10.1080/1358314X.2019.1653588

## Description

The invention relates to an intraocular lens.

In cataract treatment of an eye, an incision is conventionally made in the cornea of the eye, said incision being large enough to allow a cannula to be inserted through the incision into the eye. After the incision has been made in the cornea, the lens of the eye is broken up by phacoemulsification and then sucked out of the capsular bag of the eye. Thereafter, an intraocular lens is inserted into the capsular bag by means of an injector. The intraocular lens includes an optical body and a haptic element, wherein the haptic element fixes the optical body in the capsular bag.

The haptic element has the function of keeping the optical body as close as possible to the middle of the eye in order to generate an image of maximum quality on the retina of the eye. Moreover, the optical body should be fixed with maximum positional stability in the capsular bag. In addition, the haptic element has the function of stopping the optical body from rotating about its optical axis. This is particularly relevant when the optical body is a toric optical body by means of which cornea curvature is to be corrected, because the toric optical body, if it is arranged in the capsular bag with an incorrect orientation, leads to an imaging aberration on the retina.

Once the intraocular lens has been inserted into the capsular bag, the intraocular lens may be disposed in an incorrect position in the capsular bag. This may be caused, for example, by nonuniform fibrosis. If the intraocular lens has been disposed in the wrong position in the capsular bag, it is necessary to correct the position of the intraocular lens in a surgical intervention or even to change the intraocular lens. Remedy could be provided by an intraocular lens having a correctable position after the insertion of the intraocular lens.

EP 1 462 071 A2 discloses an accommodating intraocular lens. DE 10 2011 053 670 A1 discloses an intraocular lens. KIM DAE-YOON ET AL: "Light responsive liquid crystal soft matters: structures, properties, and applications", LIQUID CRYSTALS TODAY, vol. 28, no. 2, 3 April 2019 (2019-04-03), pages 34-45, XP055809307, GB, ISSN: 1358-314X, DOI: 10.1080/1358314X.2019.1653588 discloses a compound undergoing a reversible isomerisation. Bull James N. ET AL: "From E to Z and back again: reversible photoisomerisation of an isolated charge-tagged azobenzene", PHYSICAL CHEMISTRY CHEMICAL PHYSICS, vol. 20, no. 1, 1 January 2018 (2018-01-01), pages 509-513, XP055809255, ISSN: 1463-9076, DOI: 10.1039/C7CP07278C discloses a methyl orange anion.

The problem addressed by the invention is therefore that of providing an intraocular lens having a correctable position after it has been inserted into a capsular bag of an eye.

The intraocular lens according to the invention for introduction into the capsular bag of an eye has an optical body, a first haptic element which is secured to the optical body and has a first brace bar disposed in a first region of the intraocular lens, and a second haptic element which is secured to the optical body and has a second brace bar disposed in a second region of the intraocular lens, wherein the first region and the second region are disposed on sides of the optical body that face away from one another, and the first brace bar and the second brace bar are set up to expand after irradiation with electromagnetic radiation, wherein the first haptic element and the second haptic element are set up such that, when the intraocular lens has been introduced into the capsular bag and one of the brace bars is more intensely irradiated with the electromagnetic radiation than the other of the brace bars, the optical body moves in such a way that the optical body takes on a more centred arrangement in the capsular bag.

If the intraocular lens has been introduced into the capsular bag of an eye, the two haptic elements make contact with the capsular bag. Should the optical body not be disposed centrally in the capsular bag, daylight that strikes the intraocular lens via the pupil of the eye that is bounded by the iris of the eye is not incident on the intraocular lens in a symmetric manner, and especially not incident on the optical body in a symmetric manner. Because the daylight is not incident on the intraocular lens in a symmetric manner, one of the two brace bars is irradiated with the electromagnetic radiation in a larger region adjoining the optical body than the other of the two brace bars. Regions of the brace bars that are further removed from the optical body are disposed behind the iris and are therefore not affected by the incident daylight. Because the brace bars are set up to expand after irradiation with the electromagnetic radiation, one of the two brace bars expands more significantly than the other of the two brace bars. As a consequence, the optical body moves of its own accord within the capsular bag such that the optical body takes on a very substantially centred arrangement in the capsular bag.

It is according to the invention that the first brace bar has a first longitudinal end arranged facing toward the optical body, and a second longitudinal end arranged facing away from the optical body, and the second brace bar has a first longitudinal end arranged facing toward the optical body, and a second longitudinal end arranged facing away from the optical body, wherein the first longitudinal end of the first brace bar, the second longitudinal end of the first brace bar, the first longitudinal end of the second brace bar and the second longitudinal end of the second brace bar lie on a straight line that intersects with the optical axis of the optical body. It is preferable that the distance from the first longitudinal end of the first brace bar to the second longitudinal end of the first brace bar is equal to the distance from the first longitudinal end of the second brace bar to the second longitudinal end of the second brace bar.

It is preferable that the first brace arm and the second brace arm have a coating having a band-pass filter, a low-pass filter and/or a high-pass filter for the electromagnetic radiation. This makes it possible to manipulate the spectrum of daylight such that essentially only wavelengths that lead to expansion of the brace arm pass through the coating.

It is according to the invention that the first brace arm and the second brace arm include a substance having the structural formula

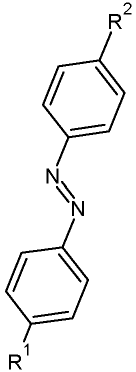

. On irradiation with the electromagnetic radiation, the compound isomerizes from the E isomer shown above to the corresponding Z isomer. This is shown in the following reaction equation, where λ symbolizes the irradiation:

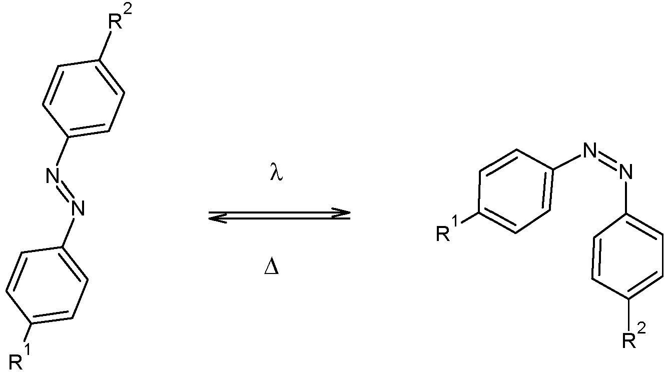

The E isomer shown on the left can more easily assume an ordered structure in the composite, and thus has a higher density than the E isomer shown on the right, which results in expansion of the brace bars on irradiation. If the irradiation ceases, the Z isomer can isomerize back to the E isomer through body heat, represented symbolically in the reaction equation by Δ. This allows brace bars to regenerate. Because the brace bars are disposed in regions of the optical body that are distant from one another, an equilibrium state will become established over time, in which the same proportion of the respective brace bar is covered by the iris in each of the two brace bars, and the same proportion of the brace bars is irradiated in each of the two brace bars.

It is according to the invention that R¹ is selected from the group of -H, -NR'R'', -NH₂, -NHCOR', -OH, and -OR', where R' and R'' are independently an aliphatic radical, an aromatic radical, a polymerizable radical, especially an acrylate, a methacrylate or a vinyl, or a polymeric radical, especially a polyacrylate, a polymethacrylate or a polyvinyl, where R² is selected from the group of -H, -NR'R'', -NH₂, -NHCOR', -OH, - OR' and 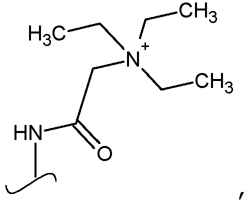 where R' and R'' are independently an aliphatic radical, an aromatic radical, a polymerizable radical, especially an acrylate, a methacrylate or a vinyl, or a polymeric radical, especially a polyacrylate, a polymethacrylate or a polyvinyl. An example of a conceivable counterion for the substance with the cation depicted above is a halide.

The first brace arm and the second brace arm preferably have a coating having a low-pass filter for the electromagnetic radiation, where the low-pass filter is set up to allow the electromagnetic radiation having wavelengths longer than a limiting wavelength to pass through to the substance with an intensity of less than 10% of an intensity of the electromagnetic radiation that strikes the low-pass filter. More preferably, the low-pass filter is set up to allow the electromagnetic radiation having wavelengths longer than the limiting wavelength to pass through to the substance with an intensity of less than 5% of the intensity of the electromagnetic radiation that strikes the low-pass filter.

A respective outer face intended for contact with the capsular bag, the first haptic element and the second haptic element is preferably structured so as to increase static friction of the outer face on the capsular bag.

According to an embodiment that does not belong the invention the first haptic element and the second haptic element each have a haptic element frame that in each case has an outer strut facing away from the optical body and a flexible inner strut that faces toward the optical body and is secured to the second longitudinal end of the brace bar, and each have a first tensile bar secured to the optical body and to the inner strut. On irradiation of the first brace bar or of the second brace bar, this expands and in so doing deforms the associated flexible inner strut. This results in indentation of the inner strut and movement of the inner strut in the direction of the associated outer strut. As a result, the first tensile bar draws the optical body in the direction of the outer strut, which results in centring of the optical body in the capsular bag. It is particularly preferable that the first haptic element and the second haptic element each have a second tensile bar that has been secured to the optical body and to the inner strut and is disposed on the side of the brace bar distant from the first tensile bar in circumferential direction. This pulls the optical body in the direction of the outer strut at two points.

The first haptic element and the second haptic element can each have a first articulation via which the first tensile bar is secured to the inner strut, and/or a second articulation via which the second tensile bar is secured to the inner strut. This allows the tensile strut to be more easily indented on irradiation of the brace bar, and reduction of mechanical stresses in the tensile bar and in the outer strut on irradiation of the brace bar.

It is especially conceivable that the outer strut is straight, with the haptic element frame having a first connecting strut and a second connecting strut that are each secured to the outer strut and the inner strut. This haptic element frame can be executed in a particularly space-saving manner. Alternatively, it is particularly conceivable that the outer strut on a side distant from the optical body has convex curvature; more particularly, the outer strut is secured to the inner strut at its two longitudinal ends. The curved outer strut fits particularly well to the capsular bag.

According to the invention, the intraocular lens has a frame that projects from the optical body on two sides of the optical body that face away from one another, wherein the second longitudinal end of the first brace bar and the second longitudinal end of the second brace bar are secured to the frame, wherein the first haptic element has a first brace element which is secured to the first longitudinal end of the first brace bar and extends as far as the side of the optical body distant from the first brace bar and projects there from the optical body further than the frame, and the second haptic element has a second brace element which is secured to the first longitudinal end of the second brace bar and extends as far as the side of the optical body distant from the second brace bar and projects there from the optical body further than the frame. On irradiation of the first brace bar or of the second brace bar, the associated brace element on the side of the optical body distant from the brace bar moves away from the optical body, which results in centring of the optical body in the capsular bag.

According to an embodiment that does not belong to the invention the intraocular lens has been set up such that, when the intraocular lens has been introduced into the capsular bag, the first brace arm and the second brace arm are shielded from direct irradiation by electromagnetic radiation that enters through a pupil of the eye, wherein the intraocular lens has a first optical fibre with an input coupling end which is disposed on a side of the optical body distant from the first brace arm and has been set up, when the intraocular lens has been introduced into the capsular bag, to capture the electromagnetic radiation that enters through the pupil of the eye, and an output coupling end that has been set up to guide the captured electromagnetic radiation onto the first brace arm, wherein the intraocular lens has a second optical fibre with an input coupling end which is disposed on a side of the optical body distant from the second brace arm and has been set up, when the intraocular lens has been introduced into the capsular bag, to capture the electromagnetic radiation that enters through the pupil of the eye, and an output coupling end that has been set up to guide the captured electromagnetic radiation onto the second brace arm. As a result, light that enters the capsular bag via the pupil does not directly strike the brace arm, but is first captured by the input coupling end, then guided by means of the optical fibre to the opposite side of the optical body, where it irradiates the brace arm, which expands, as a result of which the optical body is centred in the capsular bag.

The first longitudinal end of the brace arm is preferably secured to the optical body. The intraocular lens preferably has a clip mounted at the second longitudinal end.

The invention is elucidated in detail below with reference to the schematic drawings appended.
- Figure 1: shows a first embodiment of an intraocular lens.
- Figure 2: shows a second embodiment of the intraocular lens.
- Figure 3: shows a third embodiment of the intraocular lens.
- Figure 4: shows a fourth embodiment of the intraocular lens.

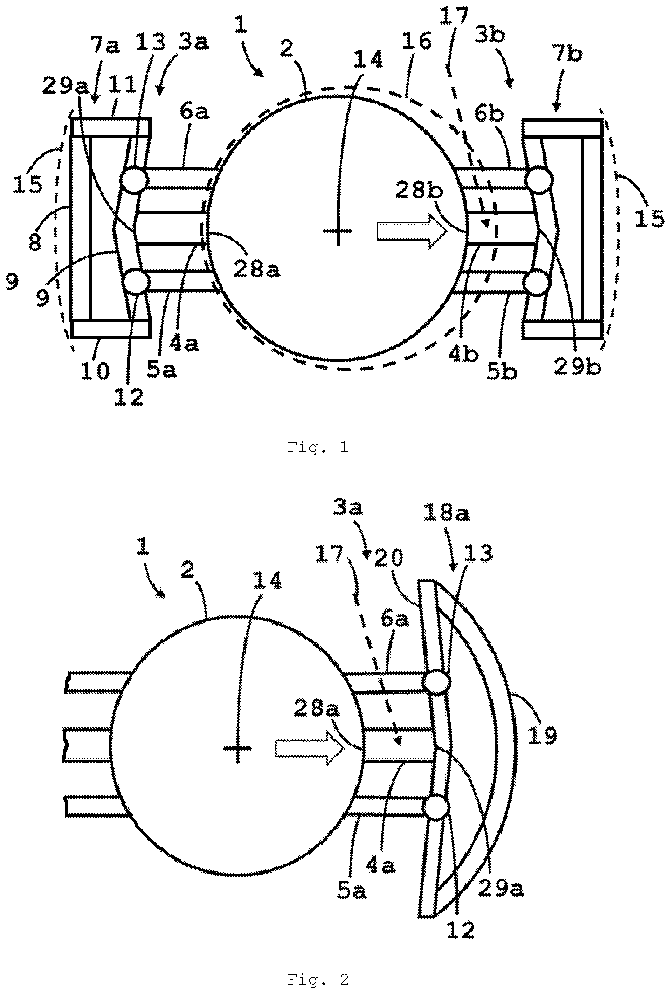

As apparent from Figures 1 to 4, an intraocular lens 1 for introduction into the capsular bag 15 of an eye has an optical body 2, a first haptic element 3a which is secured to the optical body 2 and has a first brace bar 4a disposed in a first region of the intraocular lens 1, and a second haptic element 3b which is secured to the optical body 2 and has a second brace bar 4b disposed in a second region of the intraocular lens 1, wherein the first region and the second region are disposed on sides of the optical body 2 that face away from one another, and the first brace bar 4a and the second brace bar 4b are set up to expand after irradiation with electromagnetic radiation 17, wherein the first haptic element 3a and the second haptic element 3b are set up such that, when the intraocular lens 1 has been introduced into the capsular bag 15 and one of the brace bars 4a, 4b is more intensely irradiated with the electromagnetic radiation than the other of the brace bars 4a, 4b, the optical body 2 moves in such a way that the optical body 2 takes on a more centred arrangement in the capsular bag 15.

Figures 1 to 4 additionally show that the first brace bar 4a may have a first longitudinal end 28a arranged facing toward the optical body 2, and a second longitudinal end 29a arranged facing away from the optical body 2, and the second brace bar 4b may have a first longitudinal end 28b arranged facing toward the optical body 2, and a second longitudinal end 29b arranged facing away from the optical body 2, wherein the first longitudinal end 28a of the first brace bar 4a, the second longitudinal end 29a of the first brace bar 4a, the first longitudinal end 28b of the second brace bar 4b and the second longitudinal end 29b of the second brace bar 4b may lie on a straight line that intersects with the optical axis 14 of the optical body 2. The distance from the first longitudinal end 28a of the first brace bar 4a to the second longitudinal end 29a of the first brace bar 4a here may be equal to the distance from the first longitudinal end 28b of the second brace bar 4b to the second longitudinal end 29b of the second brace bar 4b.

It is conceivable that the first brace arm and the second brace arm have a coating having a band-pass filter, a low-pass filter and/or a high-pass filter for the electromagnetic radiation.

The first brace arm 4a and the second brace arm 4b may include a substance having the structural formula

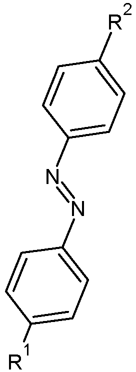

. R¹ here may be selected from the group of -H, -NR'R'', -NH₂, -NHCOR', -OH, and -OR', where R' and R'' are independently an aliphatic radical, an aromatic radical, a polymerizable radical, especially an acrylate, a methacrylate or a vinyl, or a polymeric radical, especially a polyacrylate, a polymethacrylate or a polyvinyl. R² may be selected from the group of -H, -NR'R'', -NH2, -NHCOR', -OH, -OR' and
where R' and R'' are independently an aliphatic radical, an aromatic radical, a polymerizable radical, especially an acrylate, a methacrylate or a vinyl, or a polymeric radical, especially a polyacrylate, a polymethacrylate or a polyvinyl.

The first brace arm 4a and the second brace arm 4b may have a coating having a low-pass filter for the electromagnetic radiation 17, where the low-pass filter is set up to allow the electromagnetic radiation 17 having wavelengths longer than a limiting wavelength to pass through to the substance with an intensity of less than 10% of an intensity of the electromagnetic radiation 17 that strikes the low-pass filter.

It is additionally preferable that the distance from the first longitudinal end of the first brace bar to the second longitudinal end of the first brace bar is equal to the distance from the first longitudinal end of the first brace bar to the second longitudinal end of the second brace bar.

Figure 1 shows, by way of example, an illumination spot 16 that may be the result of entry of daylight via a pupil of the eye into the capsular bag 15. What is shown is the case where the optical body 2 is not in a central arrangement in the capsular bag, and hence the intraocular lens 1 is not being symmetrically illuminated. In the example according to Figure 1, the effect of this is that a larger region of the second brace bar 4b than of the first brace bar 4a is being irradiated by the illumination spot 16. The effect of this is that the second brace bar 4b expands more significantly than the first brace bar 4a and hence becomes longer than the first brace bar 4a. Figures 1 to 4 describe four different illustrative embodiments of the intraocular lens 1 in terms of how the optical body 2 moves in such a way that the optical body takes on a more central arrangement in the capsular bag 15.

Figure 1 shows a first embodiment of the intraocular lens 1, and Figure 2 shows a second embodiment of the intraocular lens 1, wherein, in the first embodiment and the second embodiment, the first haptic element 3a and the second haptic element 3b each have a haptic element frame 7a, 7b, 18a that in each case has an outer strut 8, 19 facing away from the optical body 2 and a flexible inner strut 9, 20 that faces toward the optical body 2 and is secured to the second longitudinal end 29a, 29b of the brace bar 4a, 4b, and each have a first tensile bar 5a, 5b secured to the optical body 2 and to the inner strut 9, 20.

As is apparent from Figures 1 and 2, the first haptic element 3a and the second haptic element 3b can each have a second tensile bar 6a, 6b that has been secured to the optical body 2 and to the inner strut 9, 20 and is disposed on the side of the brace bar 4a, 4b distant from the first tensile bar 5a, 5b in circumferential direction.

Figures 1 and 2 show that the first haptic element 3a and the second haptic element 3b can each have a first articulation 12 via which the first tensile bar 5a, 5b is secured to the inner strut 9, 20, and/or a second articulation 13 via which the second tensile bar 6a, 6b is secured to the inner strut 9, 20. The first articulation 12 and/or the second articulation 13 may each be rotatable about an axis essentially parallel to the optical axis 14 of the optical body 2.

In the first embodiment of the intraocular lens 1, the outer strut 8 is straight and the haptic element frame 7a, 7b may have a first connecting strut 10 and a second connecting strut 11 that are each secured to the outer strut 8 and the inner strut 9. Figure 1 shows that one longitudinal end each of the first connecting strut 10 and the second connecting strut 11 may be disposed at one longitudinal end each of the outer strut 8, and that one longitudinal end each of the first connecting strut 10 and the second connecting strut 11 may be disposed at one longitudinal end each of the inner strut 9.

In the second embodiment of the intraocular lens 1, the outer strut 19 has convex curvature on a side distant from the optical body 2. It is conceivable here that the outer strut 19 may be secured on the inner strut 20 at its two longitudinal ends, as also shown in Figure 2.

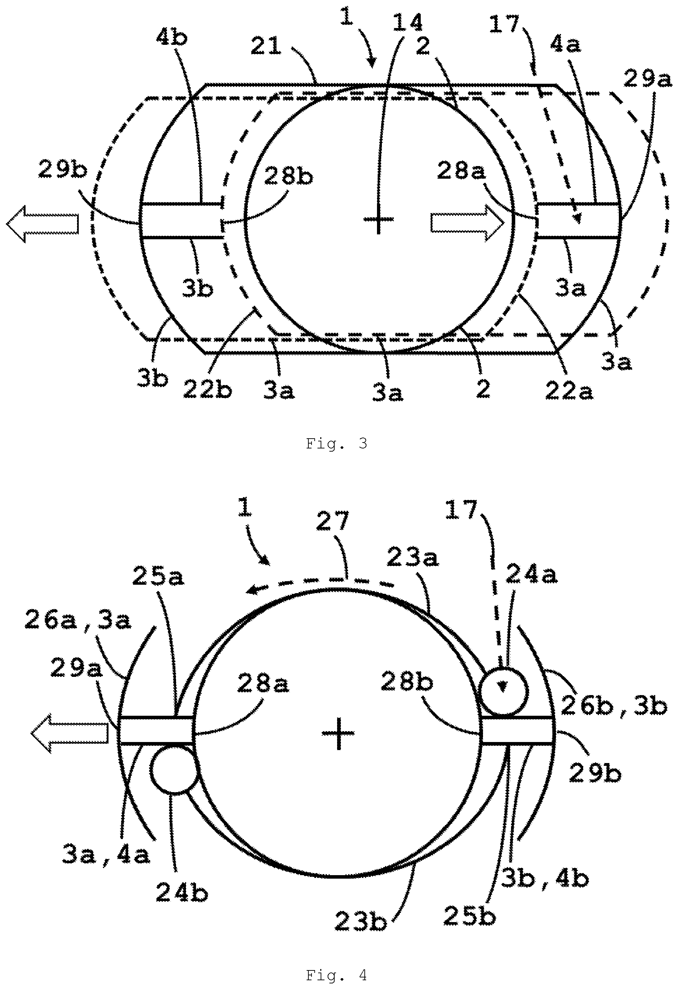

In the third embodiment of the intraocular lens, the intraocular lens 1 has a frame 21 that projects from the optical body 21 on two sides of the optical body 2 that face away from one another, wherein the second longitudinal end 29a of the first brace bar 4a and the second longitudinal end 29b of the second brace bar 4b are secured to the frame 21, wherein the first haptic element 3a has a first brace element 22a which is secured to the first longitudinal end 28a of the first brace bar 4a and extends as far as the side of the optical body 2 distant from the first brace bar 4a and projects there from the optical body 2 further than the frame 21, and the second haptic element 3b has a second brace element 22b which is secured to the first longitudinal end 28b of the second brace bar 4b and extends as far as the side of the optical body 2 distant from the second brace bar 4b and projects there from the optical body 2 further than the frame 21. Figure 3 shows that the first brace element 22a and the second brace element 22b may each have the shape of a frame. The side of the frame intended to make contact with the capsular bag 15 may have convex curvature on a side distant from the optical body 2, as also shown in Figure 3.

In the fourth embodiment of the intraocular lens 1, the intraocular lens 1 has been set up such that, when the intraocular lens 1 has been introduced into the capsular bag 15, the first brace arm 4a and the second brace arm 4b are shielded from direct irradiation by electromagnetic radiation 17 that enters through a pupil of the eye, wherein the intraocular lens 1 has a first optical fibre 23a with an input coupling end 24a which is disposed on a side of the optical body 2 distant from the first brace arm 4a and has been set up, when the intraocular lens 1 has been introduced into the capsular bag 15, to capture the electromagnetic radiation that enters through the pupil of the eye, and an output coupling end 25a that has been set up to guide the captured electromagnetic radiation onto the first brace arm 4a, wherein the intraocular lens 1 has a second optical fibre 23b with an input coupling end 24b which is disposed on a side of the optical body 2 distant from the second brace arm 4b and has been set up, when the intraocular lens 1 has been introduced into the capsular bag 15, to capture the electromagnetic radiation that enters through the pupil of the eye, and an output coupling end 25b that has been set up to guide the captured electromagnetic radiation onto the second brace arm 4b. The first brace arm 4a and the second brace arm 4b may have a shield coating that has been applied on a distal side of the two brace arms 4a, 4b and is set up to shield the two brace arms 4a, 4b from direct irradiation by the light incident through the pupil. The output coupling end 25a, 25b may be disposed on a proximal side of the two brace arms 4a, 4b and may be set up to irradiate the associated brace arm 4a, 4b from the proximal side.

As is apparent from Figure 4, the first longitudinal end 28a, 28b of the brace arm 4a, 4b may have been secured on the optical body 2. Moreover, the intraocular lens 1 may have a clip 26a, 26b that may be mounted at the second longitudinal end 29a, 29b.

Figure 4 shows that the input coupling end 24a, 24b may be arranged at an angle of the intraocular lens 1 bounded by the optical body 2 and the brace arm 4a, 4b. Alternatively or additionally, it is conceivable that the brace arm 4a, 4b has a cutout into which the input coupling end 24a, 24b has been introduced. Alternatively, it is conceivable that the input coupling end 24a, 24b is disposed in a region disposed between the optical body 2 and the brace arm 4a, 4b.

### List of reference signs

1 Intraocular lens
2 Optical body
3a First haptic element
3b Second haptic element
4a First brace bar
4b Second brace bar
5a First tensile bar of the first haptic element
5b First tensile bar of the second haptic element
6a Second tensile bar of the first haptic element
6b Second tensile bar of the second haptic element
7a First haptic element frame
7b Second haptic element frame
8 Outer strut
9 Inner strut
10 First connecting strut
11 Second connecting strut
12 First articulation
13 Second articulation
14 Optical axis
15 Capsular bag
16 Illumination spot
17 Electromagnetic radiation
18a First haptic element frame
19 Outer strut
20 Inner strut
21 Frame
22a First brace element
22b Second brace element
23a First optical fibre
23b Second optical fibre
24a Input coupling end of the first optical fibre
24b Input coupling end of the second optical fibre
25a Output coupling end of the first optical fibre
25b Output coupling end of the second optical fibre
26a First clip
26b Second clip
27 Guiding of light
28a First longitudinal end of the first brace bar
28b First longitudinal end of the second brace bar
29a Second longitudinal end of the first brace bar
29b Second longitudinal end of the second brace bar

## Claims

1. Intraocular lens for introduction into the capsular bag (15) of an eye, having an optical body (2), a first haptic element (3a) which is secured to the optical body (2) and has a first brace bar (4a) disposed in a first region of the intraocular lens (1), and a second haptic element (3b) which is secured to the optical body (2) and has a second brace bar (4b) disposed in a second region of the intraocular lens (1), wherein the first region and the second region are disposed on sides of the optical body (2) that face away from one another, and the first brace bar (4a) and the second brace bar (4b) are set up to expand after irradiation with electromagnetic radiation (17), wherein the first haptic element (3a) and the second haptic element (3b) are set up such that, when the intraocular lens (1) has been introduced into the capsular bag (15) and one of the brace bars (4a, 4b) is more intensely irradiated with the electromagnetic radiation (17) than the other of the brace bars (4a, 4b), the optical body (2) moves in such a way that the optical body (2) takes on a more centred arrangement in the capsular bag (15), **characterized in that** the first brace bar (4a) has a first longitudinal end (28a) arranged facing toward the optical body (2), and a second longitudinal end (29a) arranged facing away from the optical body (2), and the second brace bar (4b) has a first longitudinal end (28b) arranged facing toward the optical body (2), and a second longitudinal end (29b) arranged facing away from the optical body (2), wherein the first longitudinal end (28a) of the first brace bar (4a), the second longitudinal end (29a) of the first brace bar (4a), the first longitudinal end (28b) of the second brace bar (4b) and the second longitudinal end (29b) of the second brace bar (4b) lie on a straight line that intersects with the optical axis (14) of the optical body (2), wherein the intraocular lens (1) has a frame (21) that projects from the optical body (21) on two sides of the optical body (2) that face away from one another, wherein the second longitudinal end (29a) of the first brace bar (4a) and the second longitudinal end (29b) of the second brace bar (4b) are secured to the frame (21), wherein the first haptic element (3a) has a first brace element (22a) which is secured to the first longitudinal end (28a) of the first brace bar (4a) and extends as far as the side of the optical body (2) distant from the first brace bar (4a) and projects there from the optical body (2) further than the frame (21), and the second haptic element (3b) has a second brace element (22b) which is secured to the first longitudinal end (28b) of the second brace bar (4b) and extends as far as the side of the optical body (2) distant from the second brace bar (4b) and projects there from the optical body (2) further than the frame (21), wherein the first brace arm (4a) and the second brace arm (4b) include a substance having the structural formula 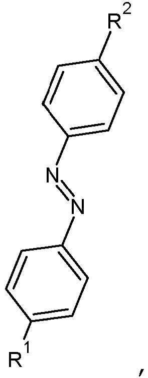 wherein R¹ is selected from the group of -H, -NR'R'', -NH₂, - NHCOR', -OH, and -OR', where R' and R'' are independently an aliphatic radical, an aromatic radical, a polymerizable radical, especially an acrylate, a methacrylate or a vinyl, or a polymeric radical, especially a polyacrylate, a polymethacrylate or a polyvinyl, where R² is selected from the group of -H, -NR'R'', -NH₂, -NHCOR', -OH, -OR' and 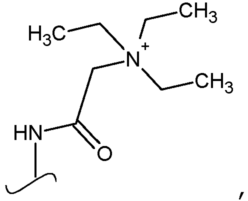 where R' and R'' are independently an aliphatic radical, an aromatic radical, a polymerizable radical, especially an acrylate, a methacrylate or a vinyl, or a polymeric radical, especially a polyacrylate, a polymethacrylate or a polyvinyl.

2. Intraocular lens according to Claim 1, wherein the first brace arm (4a) and the second brace arm (4b) have a coating having a low-pass filter for the electromagnetic radiation, wherein the low-pass filter is set up to allow the electromagnetic radiation having wavelengths longer than a limiting wavelength to pass through to the substance with an intensity of less than 10% of an intensity of the electromagnetic radiation that strikes the low-pass filter.

## Patentansprüche

1. Intraokularlinse zum Einführen in den Kapselsack (15) eines Auges, aufweisend einen optischen Körper (2), ein erstes haptisches Element (3a), das an dem optischen Körper (2) befestigt ist und einen ersten Strebenstab (4a) aufweist, der in einem ersten Bereich der Intraokularlinse (1) angeordnet ist, und ein zweites haptisches Element (3b), das an dem optischen Körper (2) befestigt ist und einen zweiten Strebenstab (4b) aufweist, der in einem zweiten Bereich der Intraokularlinse (1) angeordnet ist, wobei der erste Bereich und der zweite Bereich auf voneinander abgewandten Seiten des optischen Körpers (2) angeordnet sind und der erste Strebenstab (4a) und der zweite Strebenstab (4b) dazu eingerichtet sind, sich nach Bestrahlung mit elektromagnetischer Strahlung auszudehnen (17), wobei das erste haptische Element (3a) und das zweite haptische Element (3b) so eingerichtet sind, dass, wenn die Intraokularlinse (1) in den Kapselsack (15) eingeführt wurde und einer der Strebenstäbe (4a, 4b) mit der elektromagnetischen Strahlung (17) stärker bestrahlt wird als der andere der Strebenstäbe (4a, 4b), der optische Körper (2) sich derart bewegt, dass der optische Körper (2) eine zentriertere Anordnung in dem Kapselsack (15) annimmt, **dadurch gekennzeichnet, dass** der erste Strebenstab (4a) ein erstes Längsende (28a), das dem optischen Körper (2) zugewandt angeordnet ist, und ein zweites Längsende (29a), das dem optischen Körper (2) abgewandt angeordnet ist, aufweist und der zweite Strebenstab (4b) ein erstes Längsende (28b), das dem optischen Körper (2) zugewandt angeordnet ist, und ein zweites Längsende (29b), das dem optischen Körper (2) abgewandt angeordnet ist, aufweist, wobei das erste Längsende (28a) des ersten Strebenstabs (4a), das zweite Längsende (29a) des ersten Strebenstabs (4a), das erste Längsende (28b) des zweiten Strebenstabs (4b) und das zweite Längsende (29b) des zweiten Strebenstabs (4b) auf einer Geraden liegen, die die optische Achse (14) des optischen Körpers (2) schneidet, wobei die Intraokularlinse (1) einen Rahmen (21) aufweist, der von dem optischen Körper (21) aus auf zwei Seiten des optischen Körpers (2) vorsteht, die voneinander abgewandt sind, wobei das zweite Längsende (29a) des ersten Strebenstabs (4a) und das zweite Längsende (29b) des zweiten Strebenstabs (4b) an dem Rahmen (21) befestigt sind, wobei das erste haptische Element (3a) ein erstes Strebenelement (22a) aufweist, das an dem ersten Längsende (28a) des ersten Strebenstabs (4a) befestigt ist und sich bis zu der Seite des optischen Körpers (2), die von dem ersten Strebenstab (4a) entfernt liegt, erstreckt und dort von dem optischen Körper (2) weiter als der Rahmen (21) vorsteht, und das zweite haptische Element (3b) ein zweites Strebenelement (22b) aufweist, das an dem ersten Längsende (28b) des zweiten Strebenstabs (4b) befestigt ist und sich bis zu der Seite des optischen Körpers (2), die von dem zweiten Strebenstab (4b) entfernt liegt, erstreckt und dort von dem optischen Körper (2) weiter als der Rahmen (21) vorsteht, wobei der erste Strebenarm (4a) und der zweite Strebenarm (4b) eine Substanz mit der folgenden Strukturformel beinhalten 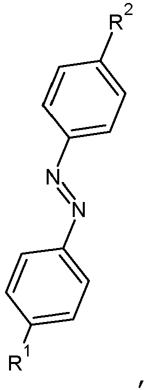 wobei R¹ ausgewählt ist aus der Gruppe von -H, -NR'R", - NH₂, -NHCOR', -OH, und -OR', wobei R' und R" auf unabhängige Weise für einen aliphatischen Rest, einen aromatischen Rest, einen polymerisierbaren Rest, insbesondere ein Acrylat, ein Methacrylat oder ein Vinyl, oder einen polymeren Rest, insbesondere ein Polyacrylat, ein Polymethacrylat oder ein Polyvinyl stehen, wobei R² ausgewählt ist aus der Gruppe -H, - NR'R", -NH₂, -NHCOR', -OH, -OR' und 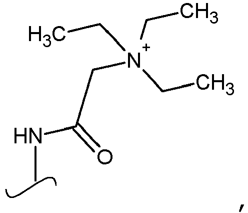 wobei R' und R" unabhängig voneinander für einen aliphatischen Rest, einen aromatischen Rest, einen polymerisierbaren Rest, insbesondere ein Acrylat, ein Methacrylat oder ein Vinyl, oder einen polymeren Rest, insbesondere ein Polyacrylat, ein Polymethacrylat oder ein Polyvinyl, stehen.

2. Intraokularlinse nach Anspruch 1, wobei der erste Strebenarm (4a) und der zweite Strebenarm (4b) eine Beschichtung mit einem Tiefpassfilter für die elektromagnetische Strahlung aufweisen, wobei der Tiefpassfilter eingerichtet ist, die elektromagnetische Strahlung mit Wellenlängen länger als eine Grenzwellenlänge mit einer Intensität von weniger als 10 % einer Intensität der elektromagnetischen Strahlung, die auf den Tiefpassfilter trifft, durchzulassen.

## Revendications

1. Lentille intraoculaire destinée à être introduite dans la poche capsulaire (15) d'un œil, comportant un corps optique (2), un premier élément haptique (3a) qui est fixé au corps optique (2) et comporte une première barre de renfort (4a) disposée dans une première zone de la lentille intraoculaire (1), et un deuxième élément haptique (3b) qui est fixé au corps optique (2) et présente une deuxième barre de renfort (4b) disposée dans une deuxième zone de la lentille intraoculaire (1), dans laquelle la première zone et la deuxième zone sont disposées sur des côtés du corps optique (2) opposés l'un à l'autre, et la première barre de renfort (4a) et la deuxième barre de renfort (4b) sont configurées pour se dilater après irradiation par un rayonnement électromagnétique (17), dans laquelle le premier élément haptique (3a) et le deuxième élément haptique (3b) sont configurés de telle sorte que, lorsque la lentille intraoculaire (1) a été introduite dans la poche capsulaire (15) et que l'une des barres de renfort (4a, 4b) est irradiée plus intensément par le rayonnement électromagnétique (17) que l'autre des barres de renfort (4a, 4b), le corps optique (2) se déplace de telle sorte que le corps optique (2) prend une disposition plus centrée dans la poche capsulaire (15), **caractérisée en ce que** la première barre de renfort (4a) présente une première extrémité longitudinale (28a) orientée vers le corps optique (2), et une deuxième extrémité longitudinale (29a) orientée à l'opposé du corps optique (2), et la deuxième barre de renfort (4b) présente une première extrémité longitudinale (28b) orientée vers le corps optique, et une deuxième extrémité longitudinale (29b) orientée à l'opposé du corps optique (2), dans laquelle la première extrémité longitudinale (28a) de la première barre de renfort (4a), la deuxième extrémité longitudinale (29a) de la première barre de renfort (4a), la première extrémité longitudinale (28b) de la deuxième barre de renfort (4b) et la deuxième extrémité longitudinale (29b) de la deuxième barre de renfort (4b) sont sur une ligne droite et sécante à l'axe optique (14) du corps optique (2), dans laquelle la lentille intraoculaire (1) présente un cadre (21) qui fait saillie du corps optique (21) sur deux côtés du corps optique (2) opposés l'un à l'autre, dans laquelle la deuxième extrémité longitudinale (29a) de la première barre de renfort (4a) et la deuxième extrémité longitudinale (29b) de la deuxième barre de renfort (4b) sont fixées au cadre (21), dans lequel le premier élément haptique (3a) comporte un premier élément de renfort (22a) qui est fixé à la première extrémité longitudinale (28a) de la première barre de renfort (4a) et s'étend aussi loin que le côté du corps optique (2) distant de la première barre de renfort (4a) et y fait saillie depuis le corps optique (2) plus loin que le cadre (21), et le deuxième élément haptique (3b) présente un deuxième élément de renfort (22b) qui est fixé à la première extrémité longitudinale (28b) de la deuxième barre de renfort (4b) et s'étend aussi loin que le côté du corps optique (2) distant de la deuxième barre de renfort (4b) et y fait saillie depuis le corps optique (2) plus loin que le cadre (21), le premier bras de renfort (4a) et le deuxième bras de renfort (4b) comprenant une substance présentant la formule structurale 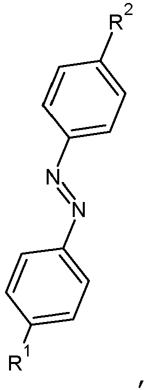 dans laquelle R¹ est choisi dans le groupe de -H, - NR'R", -NH₂, -NHCOR', -OH, et -OR', où R' et R" sont indépendamment un radical aliphatique, un radical aromatique, un radical polymérisable, notamment un acrylate, un méthacrylate ou un vinyle, ou un radical polymérique, notamment un polyacrylate, un polyméthacrylate ou un polyvinyle, où R² est choisi dans le groupe de -H, -NR'R", -NH₂, -NHCOR', -OH, -OR' et 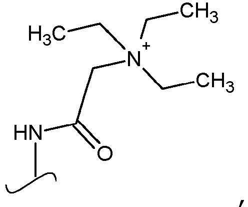 R' et R'' sont indépendamment un radical aliphatique, un radical aromatique, un radical polymérisable, notamment un acrylate, un méthacrylate ou un vinyle, un radical polymérique, notamment un polyacrylate, un polyméthacrylate ou un polyvinyle.

2. Lentille intraoculaire selon la revendication 1, dans laquelle le premier bras de renfort (4a) et le deuxième bras de renfort (4b) ont un revêtement ayant un filtre passe-bas pour le rayonnement électromagnétique, dans laquelle le filtre passe-bas est configuré pour permettre au rayonnement électromagnétique ayant des longueurs d'onde supérieures à une longueur d'onde limite de passer à travers la substance avec une intensité inférieure à 10 % de l'intensité du rayonnement électromagnétique qui frappe le filtre passe-bas.
